(19) Europäisches Patentamt
European Patent Office
Office européen des brevets

(11) Veröffentlichungsnummer: **0 161 221 B1**

(12) # EUROPÄISCHE PATENTSCHRIFT

(45) Veröffentlichungstag der Patentschrift: **10.07.91**

(21) Anmeldenummer: **85810211.4**

(22) Anmeldetag: **06.05.85**

(51) Int. Cl.5: **C07D 213/79**, C07D 213/80, C07D 471/04, C07D 491/04, //(C07D471/04,221:00,209:00), (C07D491/04,307:00,221:00)

(54) **Verfahren zur Herstellung von Pyridin-2,3-dicarbonsäurederivaten und neue 1-Amino-1,4-dihydropyridin-2,3-dicarbonsäurederivate.**

(30) Priorität: **11.05.84 CH 2337/84**

(43) Veröffentlichungstag der Anmeldung: **13.11.85 Patentblatt 85/46**

(45) Bekanntmachung des Hinweises auf die Patenterteilung: **10.07.91 Patentblatt 91/28**

(84) Benannte Vertragsstaaten: **AT BE CH DE FR GB IT LI NL SE**

(56) Entgegenhaltungen:
**EP-A- 0 041 623**
**US-A- 4 439 607**

(73) Patentinhaber: **CIBA-GEIGY AG**
**Klybeckstrasse 141**
**CH-4002 Basel(CH)**

(72) Erfinder: **Waldner, Adrian, Dr.**
**Holeeweg 39**
**D-4123 Allschwil(CH)**
Erfinder: **Roloff, Achim, Dr.**
**Waldshuterstr. 55**
**D-4310 Rheinfelden(CH)**
Erfinder: **Bellus, Daniel, Dr.**
**Unterm Schellenberg 81**
**D-4125 Riehen(CH)**

**EP 0 161 221 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von Pyridin-2,3-dicarbonsäurederivaten und neue 1-Amino-1,4-dihydropyridin-2,3-dicarbonsäurederivate, die bei der Durchführung des Verfahrens als Zwischenprodukte durchlaufen werden.

Aus der EP-A 0 041 623 sind 2-(2-Imidazolin-2-yl)-pyridin-3-carbonsäurederivate mit herbizider Wirkung bekannt, die ausgehend von entsprechenden Pyridin-2,3-dicarbonsäuren und Derivaten davon hergestellt werden können. USP 4,439,607 beschreibt ein Verfahren zur Herstellung von Pyridin-2,3-dicarbonsäureanhydriden ausgehend von entsprechenden Pyridin-2,3-dicarbonsäuren. Es steht jedoch bisher kein Verfahren zur Verfügung, das die Herstellung von Pyridin-2,3-dicarbonsäuren und Derivaten davon ausgehend von leicht zugänglichen Ausgangsmaterialien auf einfache Weise ermöglicht. Es ist das Ziel der vorliegenden Erfindung, diesem Mangel abzuhelfen und ein Verfahren bereitzustellen, nach dem Pyridin-2,3-dicarbonsäuren und ihre Derivate in einfacher Weise und in guten Ausbeuten aus billigen und leicht zugänglichen Ausgangsmaterialien hergestellt werden können.

Gegenstand der Erfindung ist ein Verfahren zur Herstellung von Pyridin-2,3-dicarbonsäurederivaten der Formel I

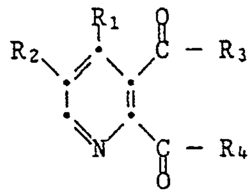

(I)

worin $R_1$ Wasserstoff, eine unsubstituierte oder durch -OH, Halogen, $C_1$-$C_4$-Alkoxy, Phenyl, Phenoxy, Cyano, Carboxyl oder $C_1$-$C_4$-Alkoxycarbonyl substituierte, lineare oder verzweigte $C_1$-$C_6$-Alkyl-, $C_1$-$C_6$-Alkylthio-oder $C_1$-$C_6$-Alkoxygruppe oder eine unsubstituierte oder durch Halogen, Cyano, $C_1$-$C_4$-Alkyl, $C_1$-$C_4$-Alkoxy, $C_1$-$C_4$-Haloalkyl oder $C_1$-$C_4$-Cyanoalkyl substituierte Phenyl- oder Phenoxygruppe bedeutet, $R_2$ die gleiche Bedeutung hat wie $R_1$ und zusätzlich Fluor, Chlor oder Brom bedeutet, $R_3$ und $R_4$ unabhängig voneinander -OH, $-NH_2$, $-NHR_5$, $-NR_5R_6$ oder $OR_7$ sind, wobei $R_5$ und $R_6$ für $C_1$-$C_6$-Alkyl, $C_5$-$C_6$-Cycloalkyl, Allyl, Methallyl, Propargyl, $C_6$-$C_{16}$-Aryl oder $C_7$-$C_{16}$-Aralkyl oder $R_5$ und $R_6$ zusammen $C_4$-$C_5$-Alkylen oder 3-Oxapentylen stehen und $R_7$ $C_1$-$C_6$-Alkyl, $C_5$-$C_6$-Cycloalkyl, Allyl, Methallyl,Propargyl, Phenyl oder Benzyl oder durch $C_1$-$C_4$-Alkyl, $C_1$-$C_4$-Alkoxy oder Halogen substituiertes Phenyl oder Benzyl darstellt, oder $R_3$ und $R_4$ zusammen -O- oder $-NR_8-$ bedeuten, wobei $R_8$ Wasserstoff, unsubstituiertes oder durch -OH, $-OR_{11}$, $-SR_{11}$, $NR_9R_{10}$, -COOH, $-COOR_7$, $-OCOR_{11}$, $-CONH_2$, $-CONHR_5$, $-CONR_5R_6$, Halogen oder Cyano substituiertes, lineares oder verzweigtes $C_1$-$C_6$-Alkyl, Allyl, Methallyl, Propargyl, unsubstituiertes oder substituiertes $C_5$-$C_6$-Cycloalkyl, Phenyl oder Benzyl oder durch $C_1$-$C_4$-Alkyl, $C_1$-$C_4$-Alkoxy oder Halogen substituiertes Phenyl oder Benzyl bedeutet, wobei $R_9$ und $R_{10}$ unabhängig voneinander für $C_1$-$C_6$-Alkyl, $C_5$-$C_6$-Cycloalkyl, Phenyl oder Benzyl oder $R_9$ und $R_{10}$ zusammen für $C_4$-$C_5$-Alkylen oder 3-Oxapentylen stehen und $R_{11}$ für $C_1$-$C_6$-Alkyl, Cyclohexyl oder Phenyl steht, das dadurch gekennzeichnet ist, dass man ein Hydrazon der Formel II

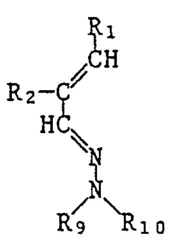

(II)

in welcher $R_9$ und $R_{10}$ die oben angegebene Bedeutung haben, in Gegenwart eines inerten Lösungsmittels mit einem Maleinsäurederivat der Formel III

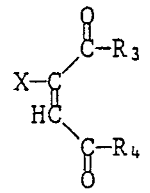

(III)

in welcher $R_3$ und $R_4$ die oben angegebene Bedeutung haben und X für Chlor oder Brom steht, zu einem 1-Amino-1,4-dihydropyridin-2,3-dicarbonsäurederivat der Formel IV

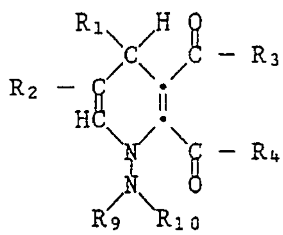

(IV)

in welcher $R_1$, $R_2$, $R_3$, $R_4$, $R_9$ und $R_{10}$ die oben angegebene Bedeutung haben, umsetzt und dieses anschliessend durch Erwärmen unter Abspaltung von $H\text{-}NR_9R_{10}$ in ein Pyridin-2,3-dicarbonsäurederivat der Formel I überführt.

In obigen Formeln enthalten $R_1$ und $R_2$ als Alkyl, Alkylthio und Alkoxy bevorzugt 1 bis 6 C-Atome. Geeignete Substituenten für diese Reste sind Hydroxy, Halogen, besonders Fluor, Chlor und Brom, $C_1$-$C_4$-Alkoxy, Phenyl, Phenoxy, Cyano, Carboxyl und $C_1$-$C_4$-Alkoxycarbonyl. Bevorzugte Substituenten sind Hydroxy und Halogen. Beispiele für diese Reste sind: Methyl, Ethyl, n-Propyl, i-Propyl, n-Butyl, i-Butyl, t-Butyl, 1-Pentyl, 3-Pentyl, 1-Hexyl, 2-Hexyl, 3-Hexyl, Methoxy, Ethoxy, n-Propoxy, i-Propoxy, n-Butoxy, i-Butoxy, t-Butoxy, Methylthio, Ethylthio, Propylthio, Butylthio, Hydroxymethyl, 2-Hydroxyethyl, Fluormethyl, Trifluormethyl, 2-Cyanoethyl, 2-Chlorethyl, Brommethyl, Benzyl, Chlorbenzyl, Methoxymethyl, Ethoxymethyl, Methoxyethyl.

Geeignete Substituenten für $R_1$ und $R_2$ als Phenyl und Phenoxy sind z.B. Halogen, wie F, Cl und Br, CN, $C_1$-$C_4$-Alkyl, $C_1$-$C_4$-Alkoxy, $C_1$-$C_4$-Haloalkyl oder $C_1$-$C_4$-Cyanoalkyl. Beispiele sind Methylphenyl, Ethylphenyl, Methoxyphenyl, Ethoxyphenyl, Chlorphenyl, Fluorphenyl, Difluorphenyl, Bromphenyl, Chlorphenoxy, Methylphenoxy, Methoxyphenoxy, Fluormethylphenyl, Difluormethylphenyl, Trifluormethylphenyl, Chlormethylphenyl, Cyanomethylphenyl, 2-Cyanoethylphenyl, Trifluormethylphenoxy und Cyanomethylphenoxy.

$R_3$ und $R_4$ sind bevorzugt -OH oder $OR_7$ und zusammen -O- oder $-NR_8$-. $R_5$ und $R_6$ enthalten als Alkyl bevorzugt 1 bis 6, besonders 1 bis 4 C-Atome. Als Cycloalkyl sind $R_5$ und $R_6$ bevorzugt Cyclopentyl oder Cyclohexyl. $R_5$ und $R_6$ zusammen sind als Alkylen bevorzugt Pentamethylen oder Tetramethylen und als Oxaalkylen bevorzugt 3-Oxapentylen. $R_5$ und $R_6$ sind als Aryl bzw. Aralkyl bevorzugt $C_6$-$C_{16}$-Aryl oder $C_7$-$C_{16}$-Aralkyl, besonders Phenyl oder Benzyl, die wie $R_1$ bis $R_3$ als Phenyl substituiert sein können.

$R_7$ kann als Alkyl linear oder verzweigt sein und bevorzugt 1 bis 6 C-Atome enthalten. $R_7$ ist als Cycloalkyl bevorzugt Cyclopentyl oder Cyclohexyl. $R_7$ als Aryl bzw. Aralkyl ist bevorzugt Phenyl oder Benzyl das substituiert sein kann, z.B. durch $C_1$-$C_4$-Alkyl, $C_1$-$C_4$-Alkoxy oder Halogen wie Fluor, Chrom oder Brom.

Geeignete Substituenten für $R_8$ sind beispielsweise -OH, $-OR_{11}$, $-SR_{11}$, worin $R_{11}$ $C_1$-$C_6$-Alkyl, Cyclohexyl oder Phenyl ist, $-NR_9R_{10}$, -COOH, $-COOR_7$, $-OCOR_{11}$, $-CONH_2$, $-CONHR_5$, $-CONR_5R_6$ und Halogen, wie z.B. Fluor, Chlor oder Brom und Cyano. $R_8$ enthält als Alkyl bevorzugt 1 bis 6 C-Atome. Beispiele sind zuvor erwähnt worden.

$R_8$ als Cycloalkyl ist bevorzugt Cyclohexyl oder Cyclopentyl. Beispiele für $R_8$ als substituierte Reste sind Hydroxymethyl, 2-Hydroxyethyl, Methoxymethyl, Methoxyethyl, Ethoxyethyl, Phenoxyethyl, Dimethylamino, Diethylamino, Methoxycarbonylmethyl, Ethoxycarbonylethyl, Propoxycarbonylmethyl, Phenoxycarbonylmethyl, Benzoyloxymethyl, Acetoyloxyethyl, Methylaminocarbonylmethyl, Dimethylaminocarbonylethyl, Chlormethyl, 2-Chlorethyl, Cyanomethyl, 2-Cyanoethyl, 2-Cyanopropyl, 2-[2-Cyano-3-methyl]butyl und 2-[2-Carbamoyl-3-methyl]butyl.

$R_9$ und $R_{10}$ als Alkyl können linear oder verzweigt sein und enthalten bevorzugt 1 bis 6, besonders 1

bis 4 C-Atome. Besonders bevorzugte Reste $R_9$ und $R_{10}$ sind Ethyl oder Methyl. Als Cycloalkyl sind $R_9$ und $R_{10}$ bevorzugt Cyclopentyl oder Cyclohexyl, als Aryl bevorzugt Phenyl und als Aralkyl bevorzugt Phenylalkyl, besonders Benzyl. In einer bevorzugten Ausführungsform sind $R_9$ Phenyl und $R_{10}$ $C_1$-$C_6$-Alkyl. $R_9$ und $R_{10}$ zusammen sind als Alkylen bevorzugt Tetramethylen oder Pentamethylen und als Oxaalkylen 3-Oxapentylen.

In einer bevorzugten Ausführungsform stehen $R_3$ und $R_4$ für einen Rest der Formel

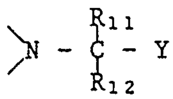 ,

worin Y für -CN oder -$CONH_2$ steht und $R_{11}$ und $R_{12}$ unabhängig voneinander für ein Wasserstoffatom oder lineares oder verzweigtes $C_1$-$C_6$-Alkyl stehen. Bevorzugt sind $R_{11}$ und $R_{12}$ lineares oder verzweigtes $C_1$-$C_4$-Alkyl.

In einer besonders bevorzugten Untergruppe stehen $R_3$ und $R_4$ für

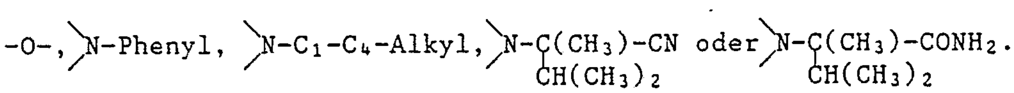

Geeignete Lösungsmittel zur Durchführung des erfindungsgemässen Verfahrens sind solche, die gegenüber den Reaktionsteilnehmern inert sind, z.B. polare, aprotische Lösungsmittel, die einzeln oder in Mischungen aus mindestens zwei Lösungsmitteln verwendet werden können. Beispiele sind: Ether wie Dibutylether, Tetrahydrofuran, Dioxan, Methylenglykol, Dimethylethylenglykol, Diethyldiethylenglykol, Dimethyltriethylenglykol, halogenierte Kohlenwasserstoffe wie Methylenchlorid, Chloroform, 1,2-Dichlorethan, 1,1,1-Trichlorethan, 1,1,2,2-Tetrachlorethan, Carbonsäureester und Lactone wie Essigsäureethylester, Propionsäuremethylester, Benzoesäureethylester, 2-Methoxyethylacetat, γ-Butyrolacton, o-Valerolacton und Pivalolacton, Carbonsäureamide und Lactame wie Formamid, Acetamid, N-Methylformamid, N,N-Dimethylformamid, N,N-Diethylformamid, N,N-Dimethylacetamid, N,N-Diethylacetamid, γ-Butyrolactam, ξ-Caprolactam, N-Methylpyrrolidon, N-Acetylpyrrolidon, N-Methylcaprolactam, Tetramethylharnstoff, Hexamethylphosphorsäuretriamid, Sulfoxide wie Dimethylsulfoxid, Sulfone wie Dimethylsulfon, Diethylsulfon, Trimethylensulfon, Tetramethylensulfon, Trimethylamin, Triethylamin, N-Methylpyrrolidin, N-Methylpiperidin, N-Methylmorpholin, substituierte Benzole wie Chlorbenzol, Nitrobenzol, Nitrile wie z.B. Acetonitril.

Die Verbindungen der Formeln II und III sind bekannt oder können nach bekannten Verfahren hergestellt werden. Sie werden bevorzugt in äquimolaren Mengen eingesetzt. Es kann aber auch ein geringer Ueberschuss der ungesättigten Hydrazone der Formel I verwendet werden.

Die Reaktion wird bevorzugt bei Temperaturen von 0° C bis 200° C, besonders 0° C bis 150° C, durchgeführt. Wenn die Temperatur mindestens 20° C, vorzugsweise mindestens 40° C beträgt, werden direkt die Verbindungen der Formel I erhalten. Durch Isomerisierung der Ausgangsverbindungen der Formel II können aber Gemische von stellungsisomeren Verbindungen der Formel I gebildet werden.

In einer bevorzugten Ausführungsform des erfindungsgemässen Verfahrens geht man zweckmässig so vor, dass man die Umsetzung eines Hydrazons der Formel II mit einem Maleinsäurederivat der Formel III zu einem 1-Amino-1,4-dihydropyridin-2,3-dicarbonsäurederivat der Formel IV in Gegenwart einer Base durchführt und anschliessend die Verbindung der Formel IV durch Behandeln mit einer Säure oder durch thermische Behandlung bei mindestens 20° C im neutralen Medium unter Abspaltung von $R_9R_{10}NH$ in eine Verbindung der Formel I überführt. $R_1$ bis $R_4$ und $R_9$ und $R_{10}$ haben die gleiche Bedeutung wie in den Formeln I und II, einschliesslich der Bevorzugungen.

Die Reaktionstemperatur beträgt in der ersten Stufe bevorzugt 0 bis 100° C, insbesondere 0 bis 50° C. Als Basen eignen sich z.B. Alkalimetallcarbonate wie Lithium-, Natrium- oder Kaliumcarbonat, und besonders organische Amine, insbesondere tertiäre Amine, die gleichzeitig als Lösungsmittel dienen können. Bevorzugt sind tertiäre Amine mit $C_1$-$C_4$-Alkylresten, wie Triäthylamin.

Die 1-Amino-1,4-dihydropyridin-2,3-dicarbonsäurederivate der Formel IV können vor der zweiten Reaktionsstufe isoliert werden. Die thermische Behandlung wird bevorzugt bei 20° C bis 150° C, insbesondere 40° C bis 100° C, vorgenommen. Geeignete Mineralsäuren sind z.B. Salzsäure, Bromwasserstoffsäure,

Jodwasserstoffsäure, Perchlorsäure oder Schwefelsäure. Wenn $R_3$ und $R_4$ hydrolyseempfindliche Gruppen darstellen, setzt man bevorzugt in einem organischen Lösungsmittel gelösten Chlorwasserstoff, Bromwasserstoff oder Chlorwasserstoff oder Jodwasserstoff ein.

Zur Herstellung von Verbindungen der Formel I ist es auch möglich, Säuren oder Säurederivate der Formel I nach bekannten Methoden in andere Verbindungen der Formel I umzuwandeln, z.B. die Anhydride in Amide, Imide, Halbester, Diester, Amidester oder Amidsäuren.

Das erfindungsgemässe Verfahren eignet sich insbesondere zur Herstellung von Pyridin-2,3-dicarbonsäurederivaten der Formel I, in welcher $R_1$ Wasserstoff oder Methyl, $R_2$ Wasserstoff, $C_1$-$C_6$-Alkyl, Fluor, Chlor, Brom, $C_1$-$C_6$-Alkoxy, $C_1$-$C_4$-Alkylthio, Phenoxy, $C_1$-$C_6$-Hydroxyalkyl, $C_1$-$C_6$-Halogenalkyl, unsubstituiertes oder durch $C_1$-$C_4$-Alkyl, $C_1$-$C_4$-Alkoxy oder Halogen substituiertes Phenyl und $R_3$ und $R_4$ einzeln je -OH oder $OR_7$ und zusammen -O- oder -$NR_8$- bedeuten. Ganz besonders eignet sich das erfindungsgemässe Verfahren zur Herstellung von Pyridin-2,3-dicarbonsäurederivaten der Formel I, in welcher $R_1$ Wasserstoff, $R_2$ $C_1$-$C_6$-Alkyl und $R_3$ und $R_4$ einzeln je -OH oder -$OR_8$ oder zusammen eine Gruppe

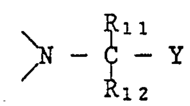

bedeuten wobei $R_8$, $R_{11}$ und $R_{12}$ jeweils die oben angegebene Bedeutung haben und Y eine Cyano- oder eine Carbamoylgruppe bedeutet.

Die durch Umsetzung eines Hydrazons der Formel II mit einem Maleinsäurederivat der Formel III in Gegenwart einer Base herstellbaren 1-Amino-1,4-dihydropyridin-2,3-dicarbonsäurederivate der Formel IV sind neue Verbindungen und bilden ebenfalls einen Gegenstand der vorliegenden Erfindung. Bevorzugt sind in diesen Verbindungen $R_1$ und $R_2$ ein Wasserstoffatom, $C_1$-$C_6$-Alkyl, $C_1$-$C_6$-Alkylthio, $C_1$-$C_6$-Alkoxy, $C_1$-$C_6$-Hydroxyalkyl, $C_2$-$C_6$-Alkoxyalkyl, $C_1$-$C_6$-Halogenalkyl, Phenyl, Phenoxy oder durch $C_1$-$C_4$-Alkyl, $C_1$-$C_4$-Alkoxy oder Halogen substituiertes Phenyl oder Phenoxy. Bevorzugt sind $R_3$ und $R_4$ -OH, -$NH_2$, -$NHR_5$, -$NR_5R_6$ oder $OR_7$, worin $R_5$ und $R_6$ $C_1$-$C_6$-Alkyl, Cyclopentyl, Cyclohexyl, Phenyl oder Benzyl oder $R_6$ und $R_5$ zusammen Trimethylen, Tetramethylen oder 3-Oxapentylen bedeuten, oder $R_3$ und $R_4$ zusammen -O- oder -$NR_8$ sind, worin $R_8$ $C_1$-$C_6$-Alkyl, $C_1$-$C_6$-Cyanoalkyl, $C_1$-$C_6$-Aminocarbonylalkyl oder Phenyl sind. $R_9$ und $R_{10}$ sind bevorzugt $C_1$-$C_6$-Alkyl, Phenyl oder zusammen Pentamethylen, Tetramethylen oder 3-Oxapentylen.

Bevorzugte Verbindungen der Formel IV sind solche, in welchen $R_1$ Wasserstoff, $R_2$ Aethyl, n-Propyl, Isopropyl oder n-Butyl, $R_9$ und $R_{10}$ Methyl und $R_3$ und $R_4$ zusammen eine Gruppe

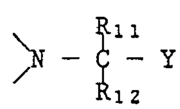

worin $R_{11}$ Methyl, $R_{12}$ Isopropyl und Y Cyano oder Carbamoyl bedeutet.

Mit dem erfindungsgemässen Verfahren gelangt man unter Verwendung einfach zugänglicher Ausgangsprodukte auf einem kurzen Syntheseweg unter milden Reaktionsbedingungen in hohen Ausbeuten zu reinen Pyridin-2,3-dicarbonsäurederviaten der Formel I. Das Verfahren ist besonders für einen technischen Prozess geeignet.

Die Verbindungen der Formel I sind wertvolle Zwischenprodukte, besonders zur Herstellung von Herbiciden, wie sie beispielsweise in der EP-A-0 041 623, EP-A-0 041 624 und EP-A-0 095 104 beschrieben sind.

Die nachfolgenden Beispiele erläutern die Erfindung näher.

Beispiel 1:

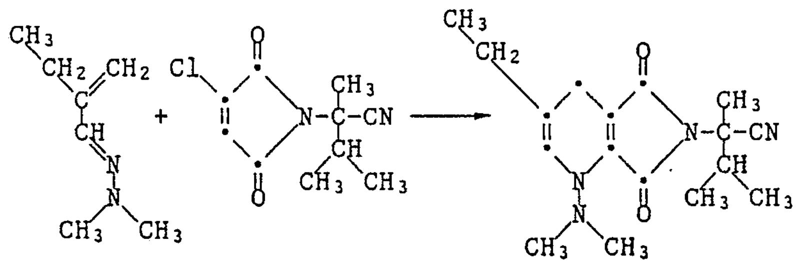

22,8 g (0,1 Mol) chloriertes Imid werden in 100 ml Acetonitril vorgelegt und mit 28 ml (0,2 Mol) Triethylamin versetzt. Bei Raumtemperatur werden 13,9 g (0,11 Mol) des Hydrazones zugetropft. Nach Abklingen der exothermen Reaktion wird noch 0,5 h bei 50 °C weitergerührt. Das Reaktionsgemisch wird filtriert und das Filtrat wird eingedampft. Der Rückstand wird über Kieselgel mit Toluol/Essigsäureethylester (16:1) chromatographiert. Man erhält 22,5 g des Dihydropyridins als rotes Oel, das beim Stehen kristallisiert. Smp. 50-51 °C.

Auf analoge Weise erhält man die folgenden Dihydropyridine:

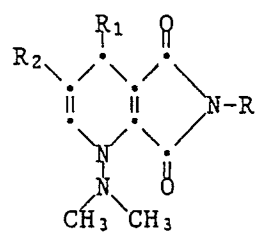

| R | $R_1$ | $R_2$ | Smp. [°C] |
|---|---|---|---|
| $C_6H_5$ | H | $C_2H_5$ | 70-72° |
| $C_6H_5$ | $CH_3$ | $CH_3$ | Oel |
| $C_6H_5$ | H | $n-C_3H_7$ | 96° |
| $C_6H_5$ | $C_2H_5$ | $CH_3$ | Oel |
| $C_6H_5$ | H | $i-C_3H_7$ | 94-95° |
| $C_6H_5$ | $CH_3$ | H | 100° |
| $C_6H_5$ | $C_6H_5$ | H | 130-132° |
| $C_6H_5$ | $C_6H_5$ | $CH_3$ | 163-165° |
| 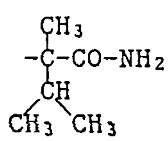 | H | $C_2H_5$ | 97-99° |
| 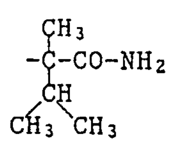 | H | $CH_3$ | |

| R | $R_1$ | $R_2$ | Smp. [°C] |
|---|---|---|---|
| 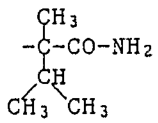 | H | $C_4H_9$ | |
| 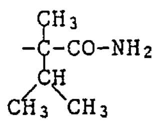 | H | $CH_2-CH_2-Cl$ | |
| 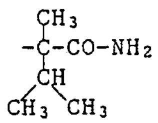 | H | $CH_2OCH_3$ | |
| 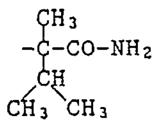 | H | $CF_3$ | |
| 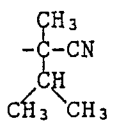 | H | $CH_3$ | |
| 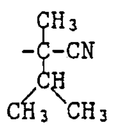 | H | $C_4H_9$ | |
| 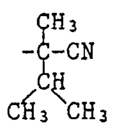 | H | $-O-CH_3$ | |
| 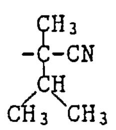 | H | $SCH_3$ | |
| 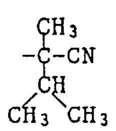 | H | 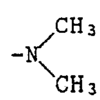 | |
| 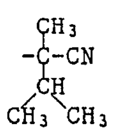 | H | Cl | |

| R | $R_1$ | $R_2$ | Smp. [°C] |
|---|---|---|---|
| 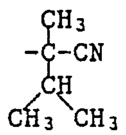 | H | $SCH_3$ | |

Beispiel 2:

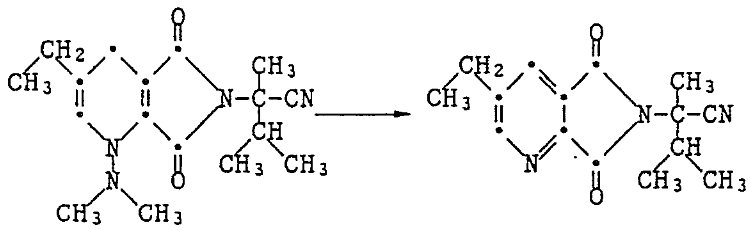

22 g Dihydropyridin werden in 100 ml Dioxan vorgelegt. Bei 8 °C wird eine Lösung von 100 ml Dioxan zugetropft, welche 10 % HCl-Gas enthält. Dann wird das Reaktionsgemisch auf Raumtemperatur erwärmt und 2 h weitergerührt. Anschliessend wird mit Soda-Lösung vorsichtig neutralisiert, mit Chloroform verdünnt, mit Wasser gewaschen, mit $MgSO_4$ getrocknet und eingedampft. Der Rückstand wird über Kieselgel wie in Beispiel 1 chromatographiert. Man erhält das Pyridin als beiges Pulver, Smp. 72-73 °C.

Statt gasförmigem HCl kann auch konz. HCl verwendet werden.

Auf analoge Weise werden folgende Verbindungen erhalten:

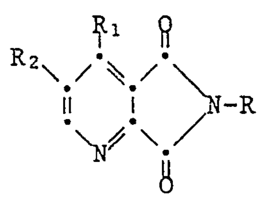

| R | $R_1$ | $R_2$ | Smp. [°C] |
|---|---|---|---|
| $C_6H_5$ | H | $C_2H_5$ | 190-191° |
| $C_6H_5$ | $CH_3$ | $CH_3$ | 165-166° |
| $C_6H_5$ | H | $n-C_3H_7$ | 149-151° |
| $C_6H_5$ | $C_2H_5$ | $CH_3$ | 174-176° |
| $C_6H_5$ | $n-C_3H_7$ | $C_2H_5$ | 94-96° |
| $C_6H_5$ | $CH_3$ | $n-C_4H_9$ | 149-151° |
| $C_6H_5$ | H | $n-C_4H_9$ | 108-110° |
| $C_6H_5$ | $n-C_3H_7$ | $CH_3$ | 122-124° |
| $C_6H_5$ | H | $i-C_3H_7$ | 124-126° |
| $C_6H_5$ | H | $CH_3$ | 244-246° |
| $C_6H_5$ | $C_6H_5$ | H | 202-203° |
| $C_6H_5$ | $p-NMe_2-C_6H_4$ | H | 206-208° |
| $C_6H_5$ | H | $(CH_2)_2CN$ | 190-191° |
| $C_6H_5$ | $CH_3$ | $n-C_4H_9$ | 149-151° |
| $C_6H_5$ | $CH_3$ | H | |
| $C_6H_5$ | $n-C_3H_7$ | H | |
| 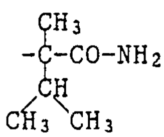 | H | Cl | |
| 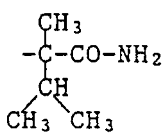 | H | $OCH_3$ | |
| 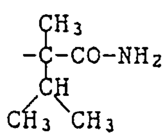 | H | $S-CH_3$ | |

| R | $R_1$ | $R_2$ | Smp. [°C] |
|---|---|---|---|
| 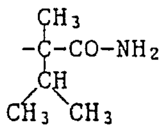 | H | $CH_2-N(CH_3)_2$ | |
| 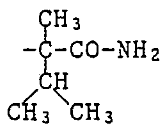 | H | $CH_2-CH_2-Cl$ | |
| 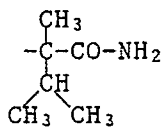 | H | $CH_2-O-CH_3$ | |
| 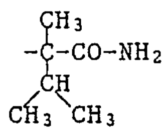 | H | $SO_2CH_3$ | |
| 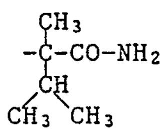 | $CH_3$ | F | 178° (Zers.) |
| $C_6H_5$ | $n-C_3H_5$ | $CH_3$ | 177-179° |
| $C_6H_5$ | H | $C_4H_9(t)$ | |
| $C_6H_5$ | H | $CH_2-CH_2Br$ | |
| $C_6H_5$ | $C_6H_5$ | $C_2H_5$ | - |
| $C_6H_5$ | $CH_3$ | 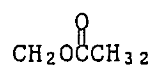 | 119-120° |
| $C_6H_5$ | H | $C_4H_9(s)$ | |
| $C_6H_5$ | H | $CH_3$ | 244-246° |
| 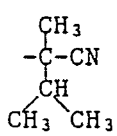 | H | $n-C_3H_7$ | 59-60° |
| 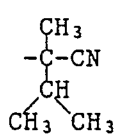 | $C_2H_5$ | $CH_3$ | 105-107° |
| 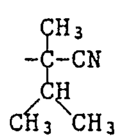 | $n-C_3H_7$ | $C_2H_5$ | 76-70° |

| R | $R_1$ | $R_2$ | Smp. [°C] |
|---|---|---|---|
| 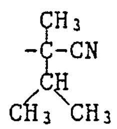 | $n-C_3H_7$ | $C_2H_5$ | 76-70° |
| 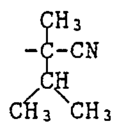 | $CH_3$ | $CH_2-CH_2Br$ | |
| 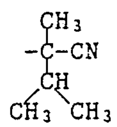 | $C_6H_5$ | $CH_3$ | 169-170° |
| 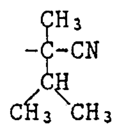 | $C_6H_5$ | $C_2H_5$ | 142-143° |
| 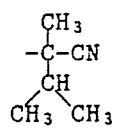 | $CH_3$ | $CH_2O\overset{O}{\overset{\|\|}{C}}CH_3$ | Oel |
| 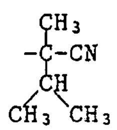 | H | $n-C_4H_9$ | Oel |
| 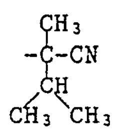 | H | $i-C_3H_7$ | 95-96° |
| 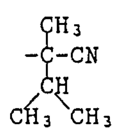 | $4-Cl-C_6H_4$ | $CH_3$ | 112-114° |
| 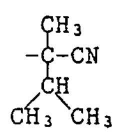 | $3-NO_2-C_6H_4$ | $CH_3$ | 164-166° |
| 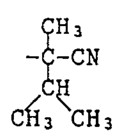 | H | $CH_3$ | 119-120° |

| R | $R_1$ | $R_2$ | Smp. [°C] |
|---|---|---|---|
| 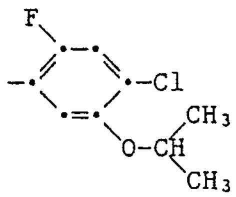 | $C_2H_5$ | $CH_3$ | 67-69° |
| 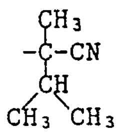 | H | $CH_2-COOC_2H_5$ | |
| 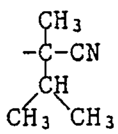 | $CH_3$ | F | Oel |
| 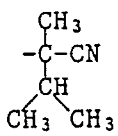 | H | 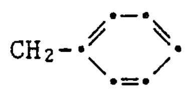 | |
| 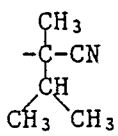 | H | $OCH_3$ | |
| 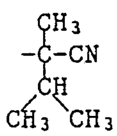 | H | Cl | |
| 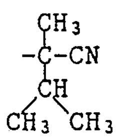 | H | $SCH_3$ | |
| 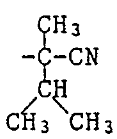 | H | $CH_2-O-CH_3$ | |
| 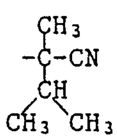 | H | $CH_2-O-CO-CH_3$ | |

Beispiel 3:

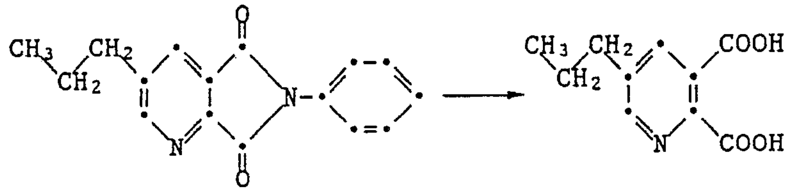

5,0 g Imid werden mit 75 ml HCl konz. unter Rückfluss erhitzt. Nach 3 1/2 h wird das Reaktionsgemisch eingedampft, in Wasser aufgenommen und erneut eingedampft. Die Pyridin-2,3-dicarbonsäure wird aus Ethanol umkristallisiert, Smp. 160-163 °C (Zers.).

Auf analoge Weise werden folgende Verbindungen erhalten:

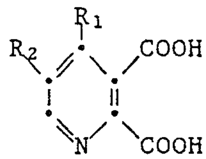

| $R_1$ | $R_2$ | Smp. [°C] |
|---|---|---|
| H | $i\text{-}C_3H_7$ | 147-148° |
| H | F | |
| H | Cl | |
| $CH_3$ | $CH_3$ | |
| $CH_3$ | $OCH_3$ | |
| F | H | |
| Cl | H | |
| Cl | $CH_3$ | |
| $C_2H_5$ | $CH_3$ | |
| $C_4H_9$ | H | |
| H | $C_4H_9(i)$ | |
| H | $C_2H_5$ | |
| H | $C_3H_7$ | |
| H | $C_4H_9(+)$ | |
| H | $OCH_3$ | |
| H | $CH_3$ | |
| H | $CH_2\text{-}CH_2\text{-}Cl$ | |
| H | $CH_2\text{-}CH_2\text{-}CN$ | |
| H | $SO_2CH_3$ | |
| H | $CH_2\text{-}O\text{-}CH_3$ | |
| H | $CH_2\text{-}OH$ | |
| H | $CH_2$- 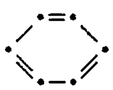 | |
| H | O- 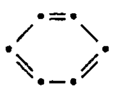 | |

Beispiel 4:

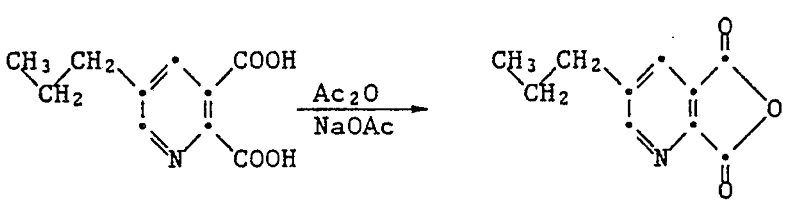


2 g 5-Propylpyridin-2,3-dicarbonsäure werden mit 25 ml Acetanhydrid und 2 g wasserfreiem Natriumacetat 1 h auf 70° C erwärmt. Nach Abdampfen des Acetanhydrids wird zwischen Wasser und Chloroform

verteilt und die organische Phase nach Trocknen über $MgSO_4$ eingedampft. Nach Kristallisation des Rückstandes mit Ether/Hexan erhält man 5-Propylpyridin-2,3-dicarbonsäureanhydrid, Smp. 42-44° C.

Auf analoge Weise wird ausgehend von 5-Isopropylpyridin-2,3-dicarbonsäure das 5-Isopropylpyridin-2,3-dicarbonsäureanhydrid vom Smp. 66-67° C und ausgehend von 5-Aethylpyridin-2,3-dicarbonsäure das 5-Aethylpyridin-2,3-dicarbonsäureanhydrid vom Smp. 71-72° C erhalten.

Beispiel 5:

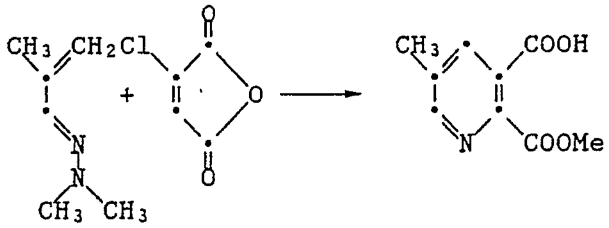

3,3 g (25 mMol) Chlormaleinsäureanhydrid, 2,8 g (25 mMol) Hydrazon und 25 ml Toluol werden 1 h bei 50° C gehalten. Nach dem Abkühlen wird das Reaktionsgemisch mit 0,1 Mol Methanol versetzt und 1 h nachgerührt. Dann wird eingedampft und der Rückstand wird über Kieselgel mit Toluol/Essigsäureethylester (1:1) chromatographiert. Man erhält 2-Methoxycarbonyl-5-methyl-3-pyridincarbonsäure, Smp. 200° C, als weisse Kristalle.

Beispiel 6:

Wird in Beispiel 6 das entsprechende 4-Methylhydrazon eingesetzt und sonst gleich verfahren, so erhält man 4-Methyl-2-methoxycarbonyl-3-pyridincarbonsäure vom Smp. 200° C (Zers.).

Beispiel 7:

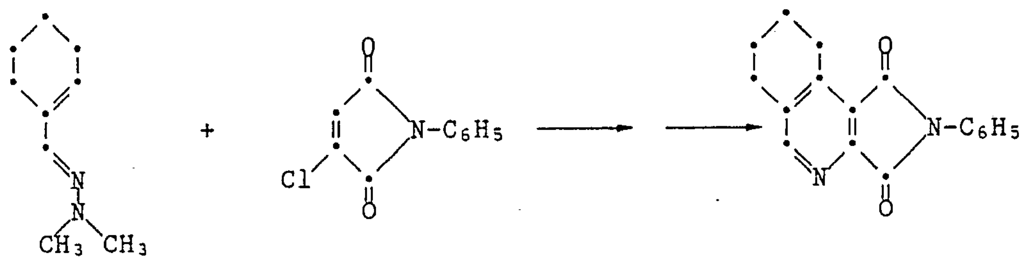

7,6 g (005 Mol) Hydrazon und 10,4 g (0.05 Mol) Imid werden mit 14 ml (0,1 Mol) Triäthylamin in 80 ml $CH_3CN$ 7,5 h auf 70° C erhitzt. Nach Abkühlen wird eingedampft und in 20 ml Dioxan suspendiert. Man tropft bei 10-20° C eine 10%ige Lösung von HCl-Gas in Dioxan zu (ca. 30 ml) und rührt während 2½ h aus. Nach Eindampfen wird in Chloroform gelöst und mit 2N Soda gewaschen. Nach Trocknen und Eindampfen wird über Kieselgel mit Toluol/Essigester chromatographiert. Man erhält 6.0 g beiges Pulver vom Smp. 142-145° C.

Auf analoge Weise werden folgende Verbindungen erhalten:

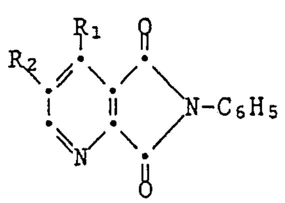

| $R_1$ | $R_2$ | Smp. [°C] |
|---|---|---|
| $CH_3$ | H | - |
| $n-C_3H_7$ | H | - |
| H | $CH_2CH_2CN$ | 190-191° |
| 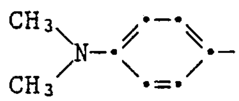 | H | 206-208° |

Beispiel 8:

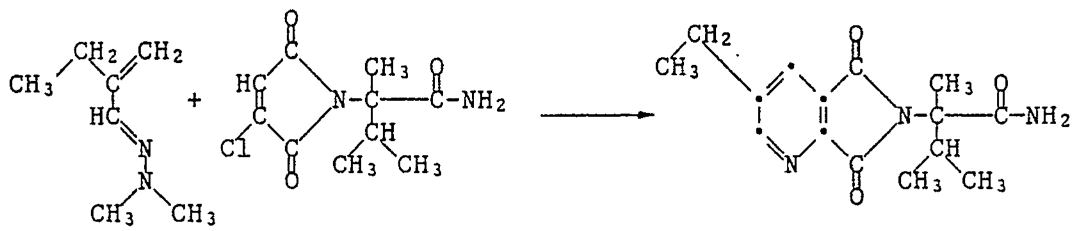

63 g Hydrazon und 122 g Chlormaleinsäureimid werden in eine Lösung von 101 g Triäthylamin in 1000 ml Acetonitril eingetragen und 8 Stunden auf 70° C erwärmt. Dann wird auf Raumtemperatur abgekühlt und filtriert und das Filtrat im Vakuum eingedampft. Der Rückstand wird in Dioxan gelöst und bei 10-20° C eine 10%ige Lösung von Chlorwasserstoff in Dioxan zugetropft. Nach beendigter Zugabe wird $1\frac{1}{2}$ Stunden bei Raumtemperatur nachgerührt. Dann wird mit Chloroform verdünnt und vorsichtig mit 2N Sodalösung neutralisiert. Dann wird die organische Phase abgetrennt, mit Wasser gewaschen, über Magnesiumsulfat getrocknet und eingedampft. Der Rückstand wird mit Aether/Hexan kristallisiert. Man erhält 105 g 3-Aethyl-5,7-dihydro-α-isopropyl-α-methyl-5,7-dioxo-6H-pyrrolo[3,4-b]pyridin-6-acetamid vom Smp. 115-117° C.

Beispiel 9:

54,2 g 3-Aethyl-5,7-dihydro-α-isopropyl-α-methyl-5,7-dioxo-6H-pyrrolo[3,4-b]pyridin-6-acetonitril werden portionenweise innert 30 Minuten in 60 ml konz. Schwefelsäure (96%ig) eingerührt. Zur Vervollständigung der Reaktion erwärmt man noch 2 Stunden auf 65° C.

Zur Aufarbeitung versetzt man mit ca. 150 g Eis, verdünnt mit 500 ml Eiswasser und puffert die Lösung durch Zugabe von 75 g Natriumacetat ab. Nach zwei Stunden Rühren bei 0° C ist die Kristallisation beendet und man filtriert das Produkt ab. Der Rückstand wird zur Reinigung in 400 ml Methylenchlorid aufgenommen, die Lösung mit Wasser gewaschen, über Natriumsulfat getrocknet und anschliessend am Rotationsverdampfer weitgehend eingeengt. Der Rückstand wird durch Zugabe von Petroläther zur Kristallisation gebracht.

Es werden 56,4 g 3-Aethyl-5,7-dihydro-α-isopropyl-α-methyl-5,7-dioxa-6H-pyrrolo[3,4-b]pyridin-6-acetamid vom Smp. 114-117° C erhalten.

## Ansprüche

**1.** Verfahren zur Herstellung von Pyridin-2,3-dicarbonsäurederivaten der Formel I

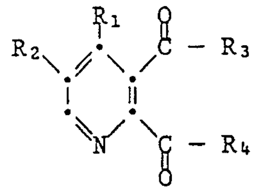

(I)

worin $R_1$ Wasserstoff, eine unsubstituierte oder durch -OH, Halogen, $C_1$-$C_4$-Alkoxy, Phenyl, Phenoxy, Cyano, Carboxyl oder $C_1$-$C_4$-Alkoxy-carbonyl substituierte, lineare oder verzweigte $C_1$-$C_6$-Alkyl-, $C_1$-$C_6$-Alkylthio-oder $C_1$-$C_6$-Alkoxygruppe oder eine unsubstituierte oder durch Halogen, Cyano, $C_1$-$C_4$-Alkyl, $C_1$-$C_4$-Alkoxy, $C_1$-$C_4$-Haloalkyl oder $C_1$-$C_4$-Cyanoalkyl substituierte Phenyl- oder Phenoxygruppe bedeutet, $R_2$ die gleiche Bedeutung hat wie $R_1$ und zusätzlich Fluor, Chlor oder Brom bedeutet, $R_3$ und $R_4$ unabhängig voneinander -OH, -$NH_2$, -$NHR$,, -$NR_5R_6$ oder $OR_7$ sind, wobei $R_5$ und $R_6$ für $C_1$-$C_6$-Alkyl, $C_5$-$C_6$-Cycloalkyl, Allyl, Methallyl, Propargyl, $C_6$-$C_{16}$-Aryl oder $C_7$-$C_{16}$-Aralkyl oder $R_5$ und $R_6$ zusammen $C_4$-$C_5$-Alkylen oder 3-Oxa-pentylen stehen und $R_7$ $C_1$-$C_6$-Alkyl, $C_5$-$C_6$-Cycloalkyl, Allyl, Methallyl, Propargyl, Phenyl oder Benzyl oder durch $C_1$-$C_4$-Alkyl, $C_1$-$C_4$-Alkoxy oder Halogen substituiertes Phenyl oder Benzyl darstellt, oder $R_3$ und $R_4$ zusammen -0- oder -$NR_8$- bedeuten, wobei $R_8$ Wasserstoff, unsubstituiertes oder durch -OH, -$OR_{11}$, -$SR_{11}$, $NR_9R_{10}$, -COOH, -$COOR_7$, -$OCOR_{11}$, -$CONH_2$, -$CONHR_5$, -$CONR_5R_6$, Halogen oder Cyano substituiertes, lineares oder verzweigtes $C_1$-$C_6$-Alkyl, Allyl, Methallyl, Propargyl, unsubstituiertes oder substituiertes $C_5$-$C_6$-Cycloalkyl, Phenyl oder Benzyl oder durch $C_1$-$C_4$-Alkyl, $C_1$-$C_4$-Alkoxy oder Halogen substituiertes Phenyl oder Benzyl bedeutet, wobei $R_9$ und $R_{10}$ unabhängig voneinander für $C_1$-$C_6$-Alkyl, $C_5$-$C_6$-Cycloalkyl, Phenyl oder Benzyl oder $R_9$ und $R_{10}$ zusammen für $C_4$-$C_5$-Alkylen oder 3-Oxapentylen stehen und $R_{11}$ für $C_1$-$C_6$-Alkyl, Cyclohexyl oder Phenyl steht, dadurch gekennzeichnet ist, dass man ein Hydrazon der Formel II

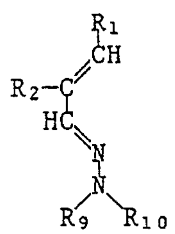

(II)

in welcher $R_9$ und $R_{10}$ die oben angegebene Bedeutung haben, in Gegenwart eines inerten Lösungsmittels mit einem Maleinsäurederivat der Formel III

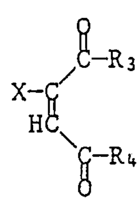

(III)

in welcher $R_3$ und $R_4$ die oben angegebene Bedeutung haben und X für Chlor oder Brom steht, zu einem 1-Amino-1,4-dihydropyridin-2,3-dicarbonsäurederivat der Formel IV

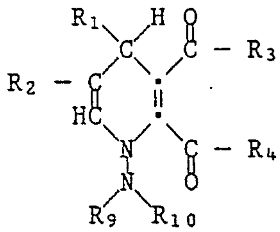

(IV)

in welcher $R_1$, $R_2$, $R_3$, $R_4$, $R_9$ und $R_{10}$ die oben angegebene Bedeutung haben, umsetzt und dieses anschliessend durch Erwärmen auf mindestens 20° C unter Abspaltung von $H\text{-}NR_9R_{10}$ in ein Pyridin-2,3-dicarbonsäurederivat der Formel I überführt.

2. Verfahren nach Anspruch 1, dadurch gekennzeichnet, dass man die Umsetzung des Hydrazons der Formel II mit dem Maleinsäurederivat der Formel III bei einer Temperatur von 0° C bis 200° C durchführt.

3. Verfahren nach Anspruch 1, dadurch gekennzeichnet, dass man die Umsetzung des Hydrazons der Formel II mit dem Maleinsäurederivat der Formel III bei einer Temperatur von 0° C bis 150° C durchführt.

4. Verfahren nach Anspruch 1, dadurch gekennzeichnet, dass man die Umsetzung des Hydrazons der Formel II mit dem Maleinsäurederivat der Formel III zum 1-Amino-1,4-dihydropyridin-2,3-dicarbonsäurederivat der Formel IV in Gegenwart einer Base durchführt und anschliessend die Verbindung der Formel IV durch Behandeln mit einer Säure oder durch thermische Behandlung bei einer Temperatur von mindestens 20° C in neutralem Medium unter Abspaltung von $R_9,R_{10}NH$ in ein Pyridin-2,3-dicarbonsäurederivat der Formel I überführt.

5. Verfahren nach Anspruch 1, dadurch gekennzeichnet, dass man ein Hydrazon der Formel II verwendet, in welchem $R_9$ und $R_{10}$ $C_1$-$C_4$-Alkyl bedeuten.

6. Verfahren nach Anspruch 4, dadurch gekennzeichnet, dass man die Umsetzung eines Hydrazons der Formel II mit einem Maleinsäurederivat der Formel III bei einer Temperatur von 0°-50° C in Gegenwart eines tertiären Amins durchführt.

7. Verfahren nach Anspruch 4, dadurch gekennzeichnet, dass man die Ueberführung des 1-Amino-1,4-dihydropyridin-2,3-dicarbonsäurederivats der Formel IV in das Pyridin-2,3-dicarbonsäurederivat der Formel I durch Einwirkung einer Mineralsäure bei einer Temperatur von 20-150° C vornimmt.

8. Verfahren nach Anspruch 4, dadurch gekennzeichnet, dass man die Ueberführung eines 1-Amino-1,4-dihydropyridn-2,3-dicarbonsäurederivats der Formel IV in ein Pyridin-2,3-dicarbonsäurederivat der Formel I durch thermische Behandlung in einem neutralen Reaktionsmedium bei einer Temperatur von 40°-100° C durchführt.

9. Verfahren nach Ansprüchen 1 und 4, dadurch gekennzeichnet, dass man in Gegenwart eines polaren, aprotischen Lösungsmittels arbeitet.

10. Verfahren nach Ansprüchen 1 und 4, dadurch gekennzeichnet, dass man ein Maleinsäurederivat der Formel III verwendet, worin $R_3$ und $R_4$ zusammen für einen Rest der Formel

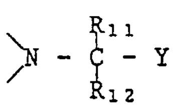

,

worin Y für -CN oder $-CONH_2$ steht und $R_{11}$ und $R_{12}$ unabhängig voneinander Wasserstoff oder $C_1$-$C_6$-

Alkyl bedeuten.

**11.** Verfahren nach Ansprüchen 1 und 4, dadurch gekennzeichnet, dass man Pyridin-2,3-dicarbonsäurederivate der Formel I herstellt, in welchen $R_1$ Wasserstoff oder Methyl, $R_2$ Wasserstoff, $C_1$-$C_6$-Alkyl, Fluor, Chlor, Brom, $C_1$-$C_6$-Alkoxy, $C_1$-$C_4$-Alkylthio, Phenoxy, $C_1$-$C_6$-Hydroxyalkyl, $C_1$-$C_6$-Halogenalkyl, unsubstituiertes oder durch $C_1$-$C_4$-Alkyl, $C_1$-$C_4$-Alkoxy oder Halogen substituiertes Phenyl und $R_3$ und $R_4$ einzeln je -OH oder -$OR_7$ oder zusammen -O- oder -$NR_8$-bedeuten.

**12.** Verfahren nach Ansprüchen 1 und 4, dadurch gekennzeichnet, dass man Pyridin-2,3-dicarbonsäurederivate der Formel I herstellt, in welchen $R_1$ Wasserstoff, $R_2$ $C_1$-$C_6$-Alkyl und $R_3$ und $R_4$ einzeln je -OH oder -$OR_7$ oder zusammen eine Gruppe

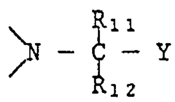

bedeuten, wobei $R_8$, $R_{11}$ und $R_{12}$ jeweils die oben angegebene Bedeutung haben und Y eine Cyano- oder eine Carbamoylgruppe bedeutet.

**13.** 1-Amino-1,4-dihydropyridin-2,3-dicarbonsäurederivate der Formel IV

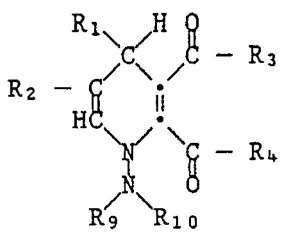

(IV)

worin $R_1$, $R_2$, $R_3$, $R_4$, $R_9$ und $R_{10}$ die unter Formel I in Anspruch 1 angegebene Bedeutung haben.

**14.** 1-Amino-1,4-dihydropyridin-2,3-dicarbonsäurederivate der Formel IV gemäss Anspruch 13 in welcher $R_1$ Wasserstoff, $R_2$ Ethyl, n-Propyl, Isopropyl oder n-Butyl, $R_9$ und $R_{10}$ je Methyl und $R_3$ und $R_4$ zusammen eine Gruppe

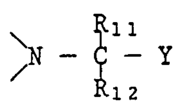

worin $R_{11}$ Methyl, $R_{12}$ Isopropyl und Y eine Cyano- oder eine Carbamoylgruppe bedeutet.

**Claims**

**1.** A process for the preparation of a pyridine-2,4-dicarboxylic acid derivative of formula I

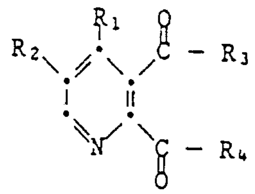

(I)

in which $R_1$ is hydrogen, or is a linear or branched $C_1$-$C_6$alkyl, $C_1$-$C_6$alkylthio or $C_1$-$C_6$alkoxy group, each of which is unsubstituted or substituted by -OH, halogen, $C_1$-$C_4$alkoxy, phenyl, phenoxy, cyano, carboxyl or $C_1$-$C_4$alkoxycarbonyl, or is a phenyl or phenoxy group, each of which is unsubstituted or substituted by halogen, cyano, $C_1$-$C_4$alkyl, $C_1$-$C_4$alkoxy, $C_1$-$C_4$haloalkyl or $C_1$-$C_4$cyanoalkyl, $R_2$ has the same meaning as $R_1$ and is additionally fluorine, chlorine or bromine, $R_3$ and $R_4$ independently of one another are -OH, $-NH_2$, $-NHR_5$, $-NR_5R_6$ or $OR_7$, in which $R_5$ and $R_6$ are $C_1$-$C_6$alkyl, $C_5$-$C_6$cycloalkyl, allyl, methallyl, propargyl, $C_6$-$C_{16}$aryl or $C_7$-$C_{16}$aralkyl, or $R_5$ and $R_6$ together are $C_4$-$C_5$alkylene or 3-oxapentylene, and $R_7$ is $C_1$-$C_6$alkyl, $C_5C_6$cycloalkyl, allyl, methallyl, propargyl, phenyl or benzyl, or is phenyl or benzyl each of which is substituted by $C_1$-$C_4$alkyl, $C_1$-$C_4$alkoxy or halogen, or $R_3$ and $R_4$ together are -O- or $-NR_8$- in which $R_8$ is hydrogen or is linear or branched C,-$C_6$alkyl which is unsubstituted or substituted by -OH, $-OR_{11}$, $-SR_{11}$, $NR_9R_{10}$, -COOH, $-COOR_7$, $-OCOR_{11}$, $-CONH_2$, $-CONHR_5$, $-CONR_5R_6$, halogen or cyano, allyl, methallyl, propargyl, unsubstituted or substituted $C_5$-$C_6$cycloalkyl, or phenyl or benzyl, or phenyl or benzyl each of which is substituted by $C_1$-$C_4$alkyl, $C_1$-$C_4$alkoxy or halogen, in which $R_9$ and $R_{10}$ independently of one another are $C_1$-$C_6$alkyl, $C_5$-$C_6$cycloalkyl, phenyl or benzyl, or $R_9$ and $R_{10}$ together are $C_4$-$C_5$alkylene or 3-oxapentylene and $R_{11}$is $C_1$-$C_6$alkyl, cyclohexyl or phenyl, which process comprises reacting a hydrazone of formula II

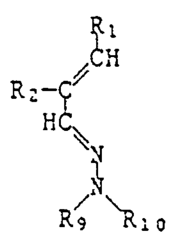

(II)

in which $R_9$ and $R_{10}$ are as defined above, with a maleic acid derivative of formula III

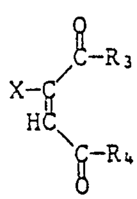

(III)

in which $R_3$ and $R_4$ are as defined above and X is chlorine or bromine, in the presence of an inert solvent, to give a 1-amino-1,4-dihydropyridine-2,3-dicarboxylic acid derivative of formula IV

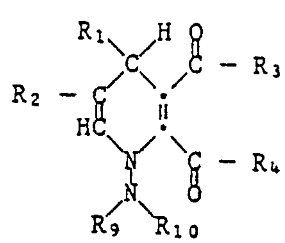

(IV)

in which $R_1$, $R_2$, $R_3$, $R_4$, $R_9$ and $R_{10}$ are as defined above, and subsequently converting the latter by heating to at least 20° C with removal of $H\text{-}NR_9R_{10}$ into a pyridine-2,3-dicarboxylic acid derivative of formula I.

**2.** A process according to claim 1, which comprises carrying out the reaction of hydrazone of formula II with the maleic acid derivative of formula III at a temperature from 0° C to 200° C.

**3.** A process according to claim 1, which comprises carrying out the reaction of the hydrazone of formula II with the maleic acid derivative of formula III at a temperature from 0° C to 150° C.

**4.** A process according to claim 1, which comprises carrying out the reaction of the hydrazone of formula II with the maleic acid derivative of formula III to give the 1-amino-1,4-dihydropyridine-2,3-dicarboxylic acid derivative of formula IV in the presence of a base and subsequently converting the compound of formula IV by treatment with an acid or by heat treatment at a temperature of at least 20° C in a neutral medium with removal of $R_9R_{10}NH$ into a pyridine-2,3-dicarboxylic acid derivative of formula I.

**5.** A process according to claim I, which comprises using a hydrazone of formula II, in which $R_9$ and $R_{10}$ are $C_1$-$C_4$alkyl.

**6.** A process according to claim 4, which comprises carrying out the reaction of a hydrazone of formula II with a maleic acid derivative of formula III at a temperature from 0° to 50° C in the presence of a tertiary amine.

**7.** A process according to claim 4, which comprises carrying out the conversion of the 1-amino-1,4-dihydropyridine-2,3-dicarboxylic acid derivative of formula IV into a pyridine-2,3-dicarboxylic acid derivative of formula I by action of mineral acid at a temperature from 20 to 150° C.

**8.** A process according to claim 4, which comprises carrying out the conversion of a 1-amino-1,4-dihydropyridine-2,3-dicarboxylic acid derivative of formula IV into a pyridine-2,3-dicarboxylic acid derivative of formula I by heat treatment in a neutral reaction medium at a temperature from 40° C to 100° C.

**9.** A process according to either of claims 1 or 4, which comprises carrying out the reaction in the presence of a polar aprotic solvent.

**10.** A process according to either of claims 1 or 4, which comprises using a maleic acid derivative of formula III, in which $R_3$ and $R_4$ together are a radical of the formula

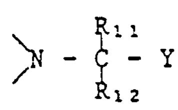 ,

in which Y is -CN or $-CONH_2$ and $R_{11}$ and $R_{12}$ independently of one another are hydrogen or $C_1$-$C_6$alkyl.

**11.** A process according to either of claims 1 or 4, which comprises preparing a pyridine-2,3-dicarboxylic acid derivative of formula I, in which $R_1$ is hydrogen or methyl, $R_2$ is hydrogen, $C_1$-$C_6$alkyl, fluorine, chlorine, bromine, $C_1$-$C_6$alkoxy, $C_1$-$C_4$alkylthio, phenoxy, $C_1$-$C_6$hydroxyalkyl, $C_1$-$C_6$haloalkyl, or phenyl which is unsubstituted or substituted by $C_1$-$C_4$alkyl, $C_1$-$C_4$alkoxy or halogen, and $R_3$ and $R_4$ individually are each -OH or $-OR_7$, or together are -O- or $-NR_8-$.

**12.** A process according to either of claims 1 or 4, which comprises preparing a pyridine-2,3-dicarboxylic acid derivative of formula I, in which $R_1$ is hydrogen, $R_2$ is $C_1$-$C_6$alkyl and $R_3$ and $R_4$ individually are each -OH or $-OR_7$ or together are a group

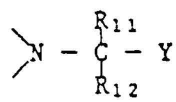

in which $R_8$, $R_{11}$ and $R_{12}$ are each as defined above and Y is a cyano or a carbamoyl group.

**13.** A 1-amino-1,4-dihydropyridine-2,3-dicarboxylic acid derivative of formula IV

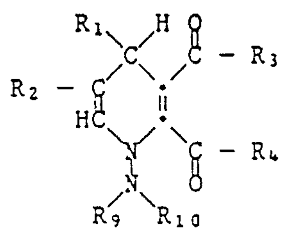

(IV)

in which $R_1$, $R_2$, $R_3$, $R_4$, $R_9$ and $R_{10}$ are as defined under formula I in claim 1.

**14.** A 1-amino-1,4-dihydropyridine-2,3-dicarboxylic acid derivative of formula IV according to claim 13, in which $R_1$ is hydrogen, $R_2$ is ethyl, n-propyl, isopropyl or n-butyl, $R_9$ and $R_{10}$ are each methyl and $R_3$ and $R_4$ together are a group

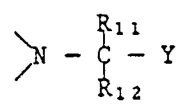

in which $R_{11}$ is methyl, $R_{12}$ is isopropyl and Y is a cyano or a carbamoyl group.

## Revendications

**1.** Procédé pour la préparation de dérivés d'acides pyridine-2,3-dicarboxyliques de formule I

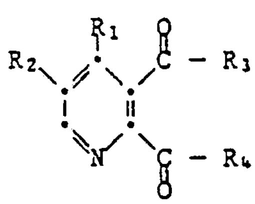

(I)

où $R_1$ représente l'hydrogène, un groupe alkyle en $C_1$ -$C_6$, alkylthio en $C_1$-$C_6$ ou alcoxy en $C_1$-$C_6$, linéaire ou ramifié, non substitué ou substitué par des radicaux -OH, halogène, alcoxy en $C_1$-$C_4$, phényle, phénoxy, cyano, carboxyle ou alcoxy($C_1$-$C_4$)-carbonyle, ou un groupe phényle ou phénoxy, non substitué ou substitué par des radicaux halogène, cyano, alkyle en $C_1$-$C_4$, alcoxy en $C_1$-$C_4$, halogénoalkyle en $C_1$-$C_4$ ou cyanoalkyle en $C_1$ -$C_4$, $R_2$ a la même signification que $R_1$ et désigne en outre le fluor, le chlore ou le brome, $R_3$ et $R_4$ représentent, indépendamment l'un de l'autre, -OH, $-NH_2$, $-NHR_5$, $-NR_5R_6$ ou $OR_7$, tandis que $R_5$ et $R_6$ représentent un alkyle en $C_1$-$C_6$, un cycloalkyle en $C_5$-$C_6$, un allyle, un méthallyle, un propargyle, un aryle en $C_6$-$C_{16}$ ou un aralkyle en $C_7$-$C_{16}$ ou $R_5$ et $R_6$ représentent ensemble un alkylène en $C_4$-$C_5$ ou un 3-oxapentylène, et $R_7$ représente un alkyle en $C_1$-$C_6$, un cycloalkyle en $C_5$-$C_6$, un allyle, un méthallyle, un propargyle, un phényle ou un benzyle ou un phényle ou benzyle substitué par un radical alkyle en $C_1$-$C_4$, alcoxy en $C_1$-$C_4$ ou halogène, ou $R_3$ et $R_4$ représentent ensemble -O- ou $-NR_8-$, tandis qur $R_8$ représente l'hydrogène, un alkyle en $C_1$-$C_6$, linéaire

ou ramifié, non substitué ou substitué par -OH, $-OR_{11}$, $-SR_{11}$, $-NR_9-R_{10}$, -COOH, $-COOR_7$, $-OCOR_{11}$, $-CONH_2$, $-CONHR_5$, $-CONR_5R_6$, halogène ou cyano, un allyle, un méthallyle, un propargyle, un cycloalkyle en $C_5$-$C_6$ non substitué ou substitué, un phényle ou un benzyle ou phényle ou benzyle substitué par un radical alkyle en $C_1$-$C_4$, alcoxy en $C_1$-$C_4$ ou halogène, $R_9$ et $R_{10}$ représentant, indépendamment l'un de l'autre, un alkyle en $C_1$-$C_6$, un cycloalkyle en $C_5$-$C_6$, un phényle ou un benzyle, ou $R_9$ et $R_{10}$ représentant ensemble un alkylène en $C_4$-$C_5$ ou du 3-oxapentylène, et $R_{11}$ désignant un alkyle en $C_1$-$C_6$, du cyclohexyle ou un phényle, qui est caractérisé en ce qu'on fait réagir une hydrazone de formule II

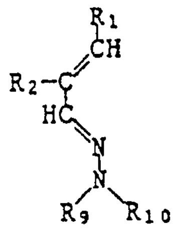

(II)

dans laquelle $R_9$ et $R_{10}$ ont la signification susdite, en présence d'un solvant inerte, avec un dérivé d'acide maléique de formule III

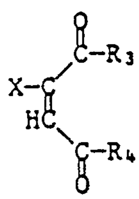

(III)

dans laquelle $R_3$ et $R_4$ ont la signification susdite et X désigne le chlore ou le brome, pour donner un dérivé d' acide 1-amino-1,4-dihydropyridine-2,3-dicarboxylique de formule IV

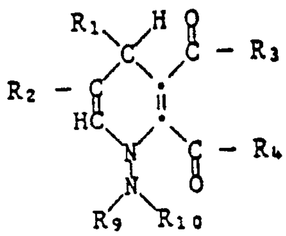

(IV)

où $R_1$, $R_2$, $R_3$, $R_4$, $R_9$ et $R_{10}$ ont la signification indiquée plus haut, et on convertit ensuite celui-ci par chauffage et élimination de $H-NR_9R_{10}$ en un dérivé d' acide pyridine-2,3-dicarboxylique de formule I.

2. Procédé selon la revendication 1, caractérisé en ce qu'on effectue la réaction de l'hydrazone de formule II avec le dérivé d'acide maléique de formule III à une température de 0° C à 200° C.

3. Procédé selon la revendication 1, caractérisé en ce qu'on effectue la réaction de l'hydrazone de formule II avec le dérivé d'acide maléique de formule III à une température de 0° C à 150° C.

4. Procédé selon la revendication 1, caractérisé en ce qu'on effectue la réaction de l'hydrazone de formule II avec le dérivé d'acide maléique de formule III pour donner le dérivé d'acide 1-amino-1,4-dihydropyridine-2,3-dicarboxylique de formule IV en présence d'une base et on convertit ensuite le composé de formule IV par traitement avec un acide ou par traitement thermique à une température d'au moins 20° C en milieu neutre avec élimination de $R_9R_{10}NH$ en un dérivé d'acide pyridine-2,3-dicarboxylique de formule I.

5. Procédé selon la revendication 1, caractérisé en ce qu'on utilise une hydrazone de formule II dans

laquelle $R_9$ et $R_{10}$ représentent un alkyle en $C_1$-$C_4$.

**6.** Procédé selon la revendication 4, caractérisé en ce qu'on effectue la réaction d'une hydrazone de formule II avec un dérivé d'acide maléique de formule III à une température de 0°-50°C en présence d'une amine tertiaire.

**7.** Procédé selon la revendication 4, caractérisé en ce qu'on réalise la transformation du dérivé d'acide 1-amino-1,4-dihydropyridine-2,3-dicarboxylique de formule IV en le dérivé d'acide pyridine-2,3-dicarboxylique de formule I sous l'action d'un acide minéral à une température de 20-150°C.

**8.** Procédé selon la revendication 4, caractérisé en ce qu'on effectue la transformation d'un dérivé d'acide 1-amino-1,4-dihydropyridine-2,3-dicarboxylique de formule IV en un dérivé d'acide pyridine-2,3-dicarboxylique de formule I par traitement thermique dans un milieu réactionnel neutre à une température de 40°-100°C.

**9.** Procédé selon l'une quelconque des revendications 1 et 4, caractérisé en ce qu'on opère en présence d'un solvant aprotique polaire.

**10.** Procédé selon l'une quelconque des revendications 1 et 4, caractérisé en ce qu'on utilise un dérivé d'acide maléique de formule III, où $R_3$ et $R_4$ représentent ensemble un reste de formule

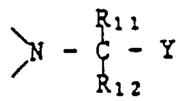 ,

où Y désigne -CN ou -$CONH_2$ et $R_{11}$ et $R_{12}$ représentent, indépendamment l'un de l'autre, l'hydrogène ou un alkyle en $C_1$-$C_6$.

**11.** Procédé selon l'une quelconque des revendications 1 et 4, caractérisé en ce qu'on prépare des dérivés d' acide pyridine-2,3-dicarboxylique de formule I dans lesquels $R_1$ est l'hydrogène ou le méthyle, $R_2$ est de l'hydrogène, un alkyle en $C_1$-$C_6$, du fluor, du chlore, du brome, un alcoxy en $C_1$-$C_6$, un alkylthio en $C_1$-$C_4$, un phénoxy, un hydroxyalkyle en $C_1$-$C_6$, un halogénoalkyle en $C_1$-$C_6$, un phényle non substitué ou substitué par des groupes alkyle en $C_1$-$C_4$,alcoxy en $C_1$-$C_4$ ou halogène et $R_3$, ainsi que $R_4$, représentent séparément chacun -OH ou -$OR_7$ ou ensemble -O- ou -$NR_8$-.

**12.** Procédé selon l'une quelconque des revendication 1 et 4, caractérisé en ce qu'on prépare des dérivés d' acide pyridine-2,3-dicarboxylique de formule I dans lesquels $R_1$ est l'hydrogène, $R_2$ est un alkyle en $C_1$-$C_6$ et $R_3$, ainsi que $R_4$, représentent séparément chacun -OH ou -$OR_7$ ou ensemble un groupe

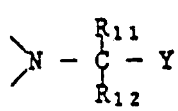

où $R_8$, $R_{11}$ et $R_{12}$ ont pour chacun la signification indiquée plus haut, tandis que Y représente un groupe cyano ou carbamoyle.

**13.** Dérivés d'acide 1-amino-1,4-dihydropyridine-2,3-dicarboxylique de formule IV

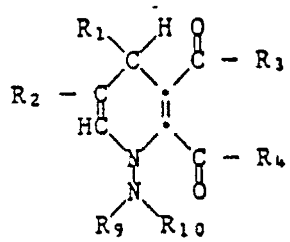

(IV)

où $R_1$, $R_2$, $R_3$, $R_4$, $R_9$ et $R_{10}$ ont la signification indiquée sous la formule I dans la revendication 1.

**14.** Dérivés d'acide 1-amino-1,4-dihydropyridine-2,3-dicarboxylique de formule IV selon la revendication 13, dans laquelle $R_1$ est l'hydrogène, $R_2$ est l'éthyle, le n-propyle, l'isopropyle ou le n-butyle, $R_9$ et $R_{10}$ sont chacun le méthyle et $R_3$ et $R_4$ représentent ensemble un groupe

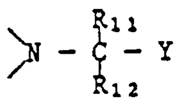

où $R_{11}$ est le méthyle, $R_{12}$ est l'isopropyle et Y est un groupe cyano ou carbamoyle.